# EUROPÄISCHE PATENTSCHRIFT

(11) **EP 0 949 937 B1**
(45) Veröffentlichungstag und Bekanntmachung des Hinweises auf die Patenterteilung: **20.08.2003**
(21) Anmeldenummer: 97945844.5
(22) Anmeldetag: 16.10.1997
(51) Int. Cl.: A61N 1/05, A61N 1/32, A61N 1/36

(54) **RETINA-IMPLANTAT**
RETINA IMPLANT
IMPLANT RETINIEN

(30) Priorität: 23.10.1996 DE 19644114; 17.02.1997 DE 19705988
(43) Veröffentlichungstag der Anmeldung: 20.10.1999
(73) Patentinhaber: Eberhard-Karls-Universität Tübingen Universitätsklinikum, 72076 Tübingen (DE)
(72) Erfinder: NISCH, Wilfried, D-72072 Tübingen (DE); STELZLE, Martin, D-72770 Reutlingen (DE); WEISS, Stefan, D-72070 Tübingen (DE); ZRENNER, Eberhard, D-72076 Tübingen (DE); STETT, Alfred, D-72768 Reutlingen (DE); GRAF, Heinz, Gerhard, D-71106 Magstadt (DE); GRAF, Michael, D-71332 Waiblingen (DE); SCHUBERT, Markus, B., D-72074 Tübingen (DE); WANKA, Harald, N., D-73630 Remshalden (DE); HIERZENBERGER, Anke, D-71067 Sindelfingen (DE)
(74) Vertreter: Witte, Alexander, Dr.-Ing.
(86) Internationale Anmeldenummer: EP9705700
(87) Internationale Veröffentlichungsnummer: WO98017343

(56) Entgegenhaltungen:
- EP-A- 0 460 320
- DE-A- 4 424 753
- DE-A- 19 529 371
- US-A- 4 628 933
- WYATT J ET AL: "OCULAR IMPLANTS FOR THE BLIND" IEEE SPECTRUM, Bd. 33, Nr. 5, 1.Mai 1996, Seiten 47, 50-53, 68/69, XP000597263

## Beschreibung

Die Erfindung betrifft ein Retina-Implantat mit einer an einer Netzhaut anliegenden Oberfläche, wobei die Oberfläche mit Elektroden zum Stimulieren von Zellen der Netzhaut versehen ist und die Elektroden von auf die Netzhaut fallendem, sichtbarem Licht derart angesteuert werden, daß ein elektrischer Stimulus von der Elektrode auf die Zelle ausgeübt wird.

Ein Retina-Implantat der vorstehend genannten Art ist aus der EP-A2-0 460 320 bekannt.

Das bekannte Implantat ist ein subretinales Implantat, das demzufolge in untere Schichten der Netzhaut implantiert werden soll. Das bekannte Implantat besteht im wesentlichen aus einem Silizium-Chip, der durch eine große Anzahl von dicht gepackten Mikro-Photodioden gebildet wird. Die photoaktive Oberfläche der Photodioden ist gegen das durch das Auge auftreffende Licht gerichtet. Die Photodioden erzeugen einen amplitudenmodulierten Strom, der die auf der Oberfläche des Implantats aufliegende Zellschicht der Netzhaut stimuliert. Auf diese Weise soll es möglich sein, Patiencen, die an unterschiedlichen Formen von Netzhautdegenerationen leiden, wieder ein Sehen oder ein besseres Sehen zu ermöglichen.

Das bekannte Implantat ist so ausgelegt, daß bereits das auftreffende Umgebungslicht ausreichen soll, um die erforderlichen Stimuli für die Zellen in der Netzhaut zu erzeugen. Eine externe Energieversorgung ist folglich nicht vorgesehen.

Aus der US-Z IEEE SPECTRUM, Mai 1996, Seiten 47 bis 53, ist ein weiteres Retina-Implantat bekannt, das jedoch als epiretinales Implantat eingesetzt werden soll. Dieses bekannte Implantat weist somit eine Elektrodenfläche auf, die vom Glaskörper her auf die Netzhaut aufgelegt werden soll, um von dort die Ganglienzellen zu stimulieren, die sich dicht unterhalb der Oberfläche der Netzhaut befinden.

Bei den epiretinalen Implantaten ist es im Gegensatz zu den subretinalen Implantaten jedoch erforderlich, die Stimuli vorab zu codieren, um auf diese Weise die biologischen Veränderungen zu kompensieren, denen die biologischen Signale auf ihrem Weg von den Photorezeptoren zu den Ganglienzellen in den unteren Schichten der Netzhaut ausgesetzt sind. Epiretinale Implantate werden daher von außen angesteuert, und zwar auf der Grundlage eines Bildes, das z.B. mit einer Videokamera oder dgl. erzeugt wurde, die bspw. als Brille für den Patienten ausgestaltet sein kann.

Aufgrund dieser systemimmanenten Unterschiede zwischen subretinalen Implantaten und epiretinalen Implantaten sind letztere bauartbedingt größer, aufwendiger und haben einen höheren Energieverbrauch.

Bei dem vorstehend erwähnten bekannten epiretinalen Implantat wird das vom Patienten zu sehende Bild mittels einer CCD-Kamera aufgenommen, die zusammen mit einem signalverarbeitenden Chip und einem Laser in einer brillenartigen Anordnung vorgesehen ist.

Bei dem Implantat selbst ist das Elektrodenarray, das zum Stimulieren der Ganglionen vorgesehen ist, als seitlich aus dem eigentlichen Implantat vorstehendes, dünnes Fähnchen ausgebildet, das von außen auf die Netzhaut aufgesetzt werden soll. Der eigentliche Implantatkörper ist demgegenüber relativ kompakt ausgebildet. Er umfaßt neben einem Stimulator-Chip ein Array von Photodioden, das gegen die Öffnung des Auges gerichtet ist. Das Photodioden-Array empfängt von dem in der brillenartigen Anordnung vorgesehenen Laser ein Lichtsignal im sichtbaren Bereich des Spektrums, das einerseits die Bildsignale enthält, andererseits aber auch zur Energieversorgung des Implantats dient.

Bei diesem bekannten epiretinalen Implantat ist somit von Nachteil, daß eine erhebliche Oberfläche im Bereich der Netzhaut mit dem Photodioden-Array abgedeckt werden muß, um die erforderliche Signalübertragung und Stromversorgung sicherzustellen. Der vom Implantat abgedeckte Teil der Netzhaut kann somit nicht zur Stimulation eingesetzt werden. Ferner ist die einstrahlbare Leistung durch die Verträglichkeitsschwelle der Netzhaut für sichtbares Licht begrenzt.

Bei dem subretinalen Implantat der eingangs zunächst genannten Art besteht hingegen das Problem, daß das Umgebungslicht mitunter nicht ausreichend sein kann, um Stimuli ausreichender Amplitude zu erzeugen, die größer sind als die Reizschwelle der zu stimulierenden Zellen der Netzhaut.

Der Erfindung liegt daher die Aufgabe zugrunde, ein Implantat der eingangs zunächst genannten Art dahingehend weiterzubilden, daß auch bei schwachem Umgebungslicht ausreichende Energie zur Verfügung steht, um Stimuli erforderlicher Amplitude erzeugen zu können. Dabei sollen diejenigen Nachteile vermieden werden, die bei epiretinalen Implantaten der ebenfalls weiter oben genannten Art auftreten und die im wesentlichen darin bestehen, daß Teile der Netzhaut von Komponenten abgedeckt werden, die nicht zur Stimulation herangezogen werden können.

Diese Aufgabe wird erfindungsgemäß bei einem Implantat der eingangs genannten Art dadurch gelöst, daß das Implantat mit einer für nicht-sichtbares Licht wirksamen, photovoltaischen Schicht versehen ist, und daß die Stimuli unter Ausnutzung der von der photovoltaischen Schicht erzeugten Spannung lokal geschaltet werden. Das Implantat ist dabei vorzugsweise großflächig mit der Schicht versehen.

Die der Erfindung zugrundeliegende Aufgabe wird auf diese Weise vollkommen gelöst.

Die Erfindung macht sich nämlich die Überlegung zunutze, daß es zur Lösung der Aufgabe zweckmäßig ist, die für die Stimulation erforderliche Versorgung mit infrarotem Licht vorzunehmen, für das die Optik ebenso wie die Netzhaut des menschlichen Auges durchlässig ist. Dadurch sind auch bei ausreichend hohen Intensitäten keine Schädigungen zu befürchten. Die im Auge zur Verfügung stehende Versorgungsleistung kann zusätzlich dadurch erhöht werden, daß die Infraroteinkopplung global, d.h. über einen möglichst großen Oberflächenbereich der Netzhaut bzw. des Implantats vorgenommen wird, während andererseits die Zellen nur lokal stimuliert werden.

Um dabei vom jeweils vorhandenen Umgebungslicht und von der Verträglichkeitsschwelle der Netzhaut für sichtbares Licht unabhängig zu sein, wird nicht-sichtbares Licht, vorzugsweise Infrarot-Licht eingesetzt, um die notwendige Energie zur Erzeugung der Stimuli bereitzustellen.

Die Energieversorgung des erfindungsgemäßen Implantats ist damit unabhängig vom jeweils vorhandenen Umgebungslicht und darüber hinaus auch unabhängig davon, ob das jeweils gesehene Bild einen hohen oder einen niedrigen Helligkeitsanteil enthält.

Bei der bevorzugten Ausgestaltung des erfindungsgemäßen Implantats sind die Elektroden von der Schicht in Draufsicht umgeben.

Diese Maßnahme hat den Vorteil, daß durch Einstellung des Oberflächenverhältnisses zwischen Elektroden und Schicht die Größe der Schicht in gewünschter Weise eingestellt werden kann. Bevorzugt ist, wenn die Elektroden etwa 10 % bis 50 % der Oberfläche der Schicht einnehmen.

Bei einer weiter bevorzugten Ausgestaltung der Erfindung umfassen die Elektroden einen optisch betätigbaren Schalter, der im geschlossenen Zustand die Spannung als Stimulus durchschaltet.

Diese Maßnahme hat den Vorteil, daß das Implantat selbsttätig gerade die Stimuli an denjenigen Punkten der Oberfläche des Implantats erzeugt, die dem tatsächlich gesehenen Bild entsprechen.

Bei einer ersten Variante dieses Ausführungsbeispiels ist der Schalter als Bereich einer weiteren Schicht ausgebildet, die bei Bestrahlung mit dem nicht-sichtbaren Licht elektrisch nicht-leitend und bei Bestrahlung mit sichtbarem Licht elektrisch leitend ist.

Diese Maßnahme hat den Vorteil, daß die Elektrodenfläche jeweils nur so groß ist, wie dies den hellen, d.h. zu stimulierenden Bereichen des Gesamtbildes entspricht. In allen dunklen Bereichen des Bildes, in denen keine Stimulierung der Zellen stattfinden muß, kann das nicht-sichtbare Licht zur zusätzlichen Energieerzeugung genutzt werden.

Bevorzugt ist bei diesem Ausführungsbeispiel die Verwendung einer Schicht, die aus einem amorphen, hydrogenisierten Silizium (a-Si:H) besteht. Es können auch Legierungen von hydrogenisiertem Silizium, also z.B. mit Kohlenstoff (a-SiC:H) oder Germanium (a-SiGe:H) verwendet werden.

Bei einer anderen Gruppe von Ausführungsbeispielen umfaßt die Elektrode als Kontaktfläche eine Schicht aus für das nicht-sichtbare Licht nicht-durchlässigem Material.

Diese Maßnahme hat den Vorteil, daß am Ort der Elektrode eine Stimulierung durch das Auftreffen von sichtbarem Licht ausgelöst werden kann, nicht jedoch durch das nicht-sichtbare Licht, das lediglich zur Energieversorgung dient.

Erfindungsgemäße Implantate können wahlweise als subretinale Implantate oder auch als epiretinale Implantat eingesetzt werden. Im letztgenannten Fall ist es lediglich erforderlich, das Implantat für sichtbares Licht durchlässig zu machen.

Bei weiteren Gruppen von Ausführungsbeispielen weist das Implantat ein Substrat mit Durchgangsöffnungen auf.

Diese Maßnahme hat den Vorteil, daß insbesondere bei einem subretinalen Implantat die Versorgung der Netzhaut mit Nährstoffen verbessert wird, weil diese durch die Durchgangsöffnungen hindurch stattfinden kann.

Die Durchgangsöffnungen können dabei alternativ in einem starren Substrat vorgesehen sein, es ist aber auch möglich, das Substrat flexibel oder als Netz aus einem flexiblen Trägermaterial auszubilden.

Im letztgenannten Fall ergibt sich nicht nur der Vorteil einer besseren Versorgung der Netzhaut. Darüber hinaus erzielt man den Vorteil, daß infolge der Verwendung von flexiblem Material eine bessere Anpassung des Implants an die Form der Netzhaut möglich ist.

Weitere Vorteile ergeben sich aus der Beschreibung und der beigefügten Zeichnung.

Ausführungsbeispiele der Erfindung sind in der Zeichnung dargestellt und werden in der nachfolgenden Beschreibung näher erläutert. Es zeigen:
- Fig. 1: einen äußerst schematisierten Querschnitt durch ein Auge, dessen Netzhaut mit einem erfindungsgemäßen Implantat versehen ist;
- Fig. 2: in stark vergrößertem Maßstab einen seitlichen Schnitt durch einen Teil eines Implantats in subretinaler Anordnung; und
- Fig. 3: eine Darstellung, ähnlich Fig. 2, jedoch für ein abgewandeltes Ausführungsbeispiel der Erfindung.

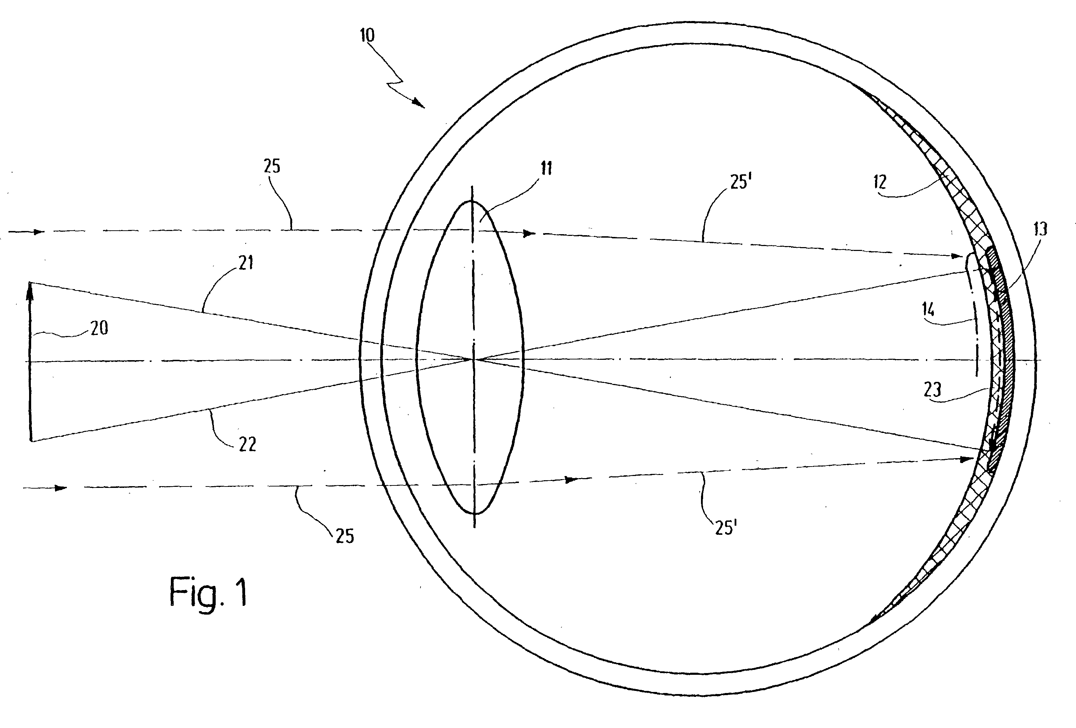

In Fig. 1 bezeichnet 10 insgesamt ein Auge, bspw. ein menschliches Auge. Im Auge 10 sind schematisch eine Linse 11 sowie eine Netzhaut 12 angedeutet.

Bei 13 ist ein subretinales Implantat zu erkennen, das sich in unteren Schichten der Netzhaut 12 befindet. Strichpunktiert ist bei 14 hingegen ein epiretinales Implantat angedeutet, das auf die Oberfläche der Netzhaut aufgesetzt wird.

Ein Gegenstand 20 wird in der üblichen Weise durch die Linse 11 auf der Netzhaut 12 abgebildet, wie mit Strahlen 21, 22 und einem auf dem Kopf stehenden Bild 23 angedeutet.

Mit 25 ist in Fig. 1 ein Bündel von Strahlen eines nicht-sichtbaren Lichtes, insbesondere eines Infrarot-Lichtes, angedeutet. Das Lichtbündel 25 ist so ausgelegt, daß es das Implantat 13 bzw. 14 vollflächig trifft. Hierzu wird das Lichtbündel 25 durch die Linse 11 lediglich geringfügig verändert, wie mit 25' im Auge 10 angedeutet. Eine Fokussierung des Lichtbündels 25 soll vermieden werden, um lokale Erwärmungen im Augeninneren zu verhindern. Das Lichtbündel 25 ist nur schematisch angedeutet. Wegen der Wellenlänge des Infrarotlichtes (λ < 1 µm) muß die Infrarot-Lichtquelle nahe vor dem äußeren Brennpunkt der Linse 11 liegen.

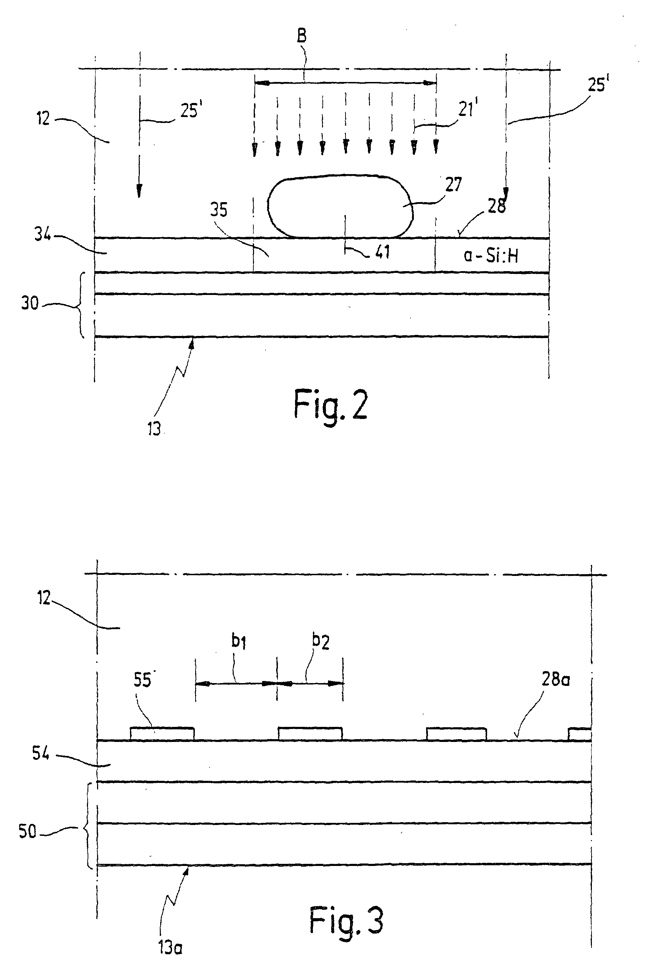

In Fig. 2 ist das Implantat 13 im wesentlichen unterhalb der Netzhaut 12, also in subretinaler Lage, zu erkennen. Mit 27 ist eine einzelne Zelle der Netzhaut 12 angedeutet. Die Zelle 27 wird an ihrer Unterseite von einer Oberfläche 28 des Implantats 13 kontaktiert.

Der Schichtaufbau des Implantats 13 ist wie folgt :

Die untersten Schichten werden durch eine Infrarot-Diode 30 gebildet. Die Infrarot-Diode 30 kann wahlweise aus kristallinem Silizium bestehen und z.B. als P-N- oder N-P-Übergang ausgebildet sein. Es sind aber auch dreischichtige Konfigurationen N-I-P oder P-I-N (jeweils von oben nach unten) möglich. Die Oberfläche 28 des Implantats 13 wird durch eine amorphe Schicht 34 aus hydrogenisiertem Silizium (a-Si:H) gebildet. Derartige Schichten sind an sich bekannt. Sie enthalten bspw. zwischen 2 % und 10 % Wasserstoff im Silizium. Die Infrarot-Diode 30 kann aus einer Legierung mit hydrogenisiertem Silizium, z.B. a-SiGe:H oder aus mikrokristallinem Silizium in einer Struktur bestehen.

Wichtig ist in diesem Zusammenhang vor allem, daß unterhalb derjenigen Ebene, durch die ein Stimulus ausgeübt werden soll, also im dargestellten Beispiel unterhalb der amorphen Schicht 34 eine relativ hochdotierte Schicht angeordnet ist, die eine genügend hohe Querleitfähigkeit aufweist, um lateral Strom zum Stimulationsort leiten zu können.

Mit 25' sind in Fig. 2 wiederum die Lichtstrahlen des infraroten Lichtes bezeichnet, die vollflächig auf die Oberfläche 28 auftreffen. Die amorphe Schicht 34 ist für das infrarote Licht durchlässig; sie ist nur schwach oder überhaupt nicht dotiert. Solange nur infrarotes Licht auf die amorphe Schicht 34 auftrifft, ist deren Leitfähigkeit sehr niedrig, sie wirkt also praktisch wie ein Isolator.

Mit B ist in Fig. 2 angedeutet, daß bei dem speziell gesehenen Bild ein bestimmter Bereich B mit sichtbarem Licht 21' bestrahlt wird.

Wenn sichtbares Licht 21' auf die amorphe Schicht 34 fällt, erhöht sich deren Leitfähigkeit schlagartig um mehrere Größenordnungen.

Dies hat zur Folge, daß in einem lokalen Bereich 35 der amorphen Schicht 34, die in der schematisierten Darstellung gemäß Fig. 2 gerade der Breite B entspricht, ein Bereich von sehr guter elektrischer Leitfähigkeit entsteht. Der Bereich 35 wirkt daher wie ein lichtgesteuerter elektrischer Schalter.

Infolge des auftreffenden infraroten Lichtes 25' ist die Infrarot-Diode 30 in der Lage, einen Strom zu liefern, der wesentlich höher als bei rein sichtbarer Anregung sein kann. Wenn nun im Bereich 35, wie oben beschrieben, "durchgeschaltet" wird, kommt es daher zu einer Stimulierung der auf dem Bereich 35 aufliegenden Zelle 27, wie mit einem Pfeil 41 angedeutet. Es wurde bereits erwähnt, daß unterhalb dieses Ortes (Pfeil 41) eine relativ hochdotierte Schicht angeordnet sein sollte, deren genügend hohe Querleitfähigkeit eine laterale Stromleitung zum Stimulationsort gestattet.

Das Potential dieser Stimulierung 41 bemißt sich dabei nicht nur an der in dem Bereich B aufgetroffenen optischen Energie. Vielmehr kann die gesamte Fläche, die zum jeweiligen Zeitpunkt nicht von sichtbarem Licht beleuchtet wird, ausgenutzt werden. Dies wiederum hat zur Folge, daß insbesondere bei schwacher Beleuchtung lokal sehr große Stimuli erzeugt werden können.

Bei der in Fig. 3 gezeigten Variante besteht das Implantat 13a ebenfalls aus einer mehrschichtigen Anordnung. Die unterste Schicht ist eine Infrarot-Diode 50. Darüber ist eine I-Schicht 54 angeordnet, auf deren Oberfläche 28a sich diskret verteilte Elektroden 55 befinden. Die Infrarot-Diode 50 entspricht in ihrer Ausführung im wesentlichen der weiter oben zu Fig. 2 beschriebenen Infrarot-Diode 30.

Die Elektroden 55 haben eine Überdeckung der Oberfläche, die vorzugsweise im Bereich von 10 % bis 20 % liegt. Aber auch höhere Überdeckungsgrade von 50 % und mehr sind bei bestimmten Anwendungsfällen denkbar.

Die Elektroden 55 sind vorzugsweise aufgedampfte Goldelektroden, die spektral selektiv durchlässig für sichtbares Licht sind. Für infrarotes Licht sind die Elektroden 55 in diesem Fall dann nicht-durchlässig.

Bei der Anordnung gemäß Fig. 3 wird somit die Energieversorgung über denjenigen Flächenanteil der Oberfläche 28a bewirkt, der in Fig. 3 mit b₁ gekennzeichnet ist. Demzufolge wird die Auflösung der Bilddarstellung durch denjenigen Oberflächenanteil bestimmt, der mit b₂ angedeutet ist.

Das auftreffende Infrarot-Licht erzeugt in der Infrarot.-Diode 50 Ladungsträger, so daß ausreichend Energie bereitgestellt wird, um beim Auftreffen von sichtbarem Licht auf eine oder mehrere Elektroden 55 dort einen Stimulus zu erzeugen. Hierzu wirkt die Schicht 54 als elektronischer Schalter, der optisch ansteuerbar ist, wenn sichtbares Licht auf und durch die Kontakte 55 fällt. Weitere Einzelheiten dazu sind in der nicht-vorveröffentlichten deutschen Patentanmeldung 195 29 371.1 vom 10.08.1995 beschrieben, auf deren Offenbarung ausdrücklich verwiesen wird.

Zur Energieversorgung kann z.B. eine Infrarot-Diode verwendet werden, die vom Patienten an einer Brille oder dgl., d.h. nahe vor der Augenlinse, getragen wird. Die Infrarot-Diode ist auf das Implantat 13 bzw. 13a gerichtet. Bei einem Durchmesser des Implantats von z.B. 3,0 mm wird eine Ausgangsleistung der Infrarot-Diode von 0,35 mW für ausreichend gehalten.

Bei subretinalen Implantaten kann unter bestimmten Umständen ein Problem bei der biologischen Versorgung der Netzhaut auftreten. Dies liegt daran, daß bei subretinaler Implantation eine bestimmte Fläche der Netzhaut "von unten" durch den Implantatkörper abgedichtet ist.

Um diese Probleme zu lösen, sind im Rahmen der vorliegenden Erfindung, aber unabhängig davon, verschiedene Lösungsansätze denkbar:

Bei einer ersten Variante werden vernetzte Retina-Implantate eingesetzt. Das Substrat des Implantats besteht daher aus einem netzartigen Gebilde. An den Knoten des Netzes befinden sich Photodioden. Das Netz selbst besteht aus einem mehr oder weniger flexiblen Trägermaterial. Dieses kann sich aufgrund dessen auch besser dem Augenhintergrund anpassen, als dies bei starren Implantaten der Fall ist.

Um derartige netzartige und/oder flexible Implantate herzustellen, werden die Photodioden auf einer SOI-Schicht (Silicon-On-Insulator) gefertigt, d.h. auf einer flexiblen, organischen Folie. Sie werden dann durch Ätzprozesse getrennt und mit einer Lack- bzw. Polyimidschicht beschichtet, die im Anschluß daran strukturiert wird. Diese strukturierte Schicht kann auch als Maske des Ätzprozesses verwendet werden.

Bei anderen Ausführungsbeispielen mit starrem Implantat werden Substrate als durchgehende Siliziumschichten ausgebildet. Diese Substrate können nun ebenfalls mit Löchern versehen werden, durch die die Netzhaut versorgt werden kann.

Zur Herstellung derartiger Implantate wird bei der Herstellung der Photodioden ein zweistufiger Ätzprozeß durchgeführt. Dabei werden Löcher in der Siliziumoberfläche mit einer Tiefe von z.B. 20 µm hergestellt. Beim späteren Rückpolieren der Chips als Implantate werden diese Löcher von der Rückseite geöffnet und dienen als Versorgungskanäle für die Netzhaut.

## Patentansprüche

1. Retina-Implantat (13,14) mit einer an einer Netzhaut (12) anlegbaren Oberfläche (28), wobei die Oberfläche (28) mit Elektroden (35; 55) zum Stimulieren von Zellen (27) der Netzhaut (12) versehen ist und die Elektroden (35; 55) von auf die Netzhaut (12) fallendem, sichtbarem Licht (21') derart angesteuert werden, daß ein elektrischer Stimulus (41) von den Elektroden (35; 55) auf die Zellen (27) ausübbar ist, **dadurch gekennzeichnet, daß** das Implantat (13; 14) mit einer für nicht-sichtbares Licht (25') wirksamen, photovoltaischen Schicht (30;50) versehen ist, und daß die Stimuli (41) unter Ausnutzung der von der photovoltaischen Schicht (30; 50) erzeugten Spannung lokal schaltbar sind.

2. Retina-Implantat nach Anspruch 1, **dadurch gekennzeichnet, daß** das Implantat (13; 14) großflächig mit der Schicht (30; 50) versehen ist.

3. Retina-Implantat nach Anspruch 1 oder 2, **dadurch gekennzeichnet, daß** die Elektroden (35; 55) von der Schicht (30; 50) in Draufsicht umgeben sind.

4. Retina-Implantat nach Anspruch 3, **dadurch gekennzeichnet, daß** die Elektroden (55) etwa 10% bis 50% der Oberfläche der Schicht (50) einnehmen.

5. Retina-Implantat nach einem oder mehreren der Ansprüche 1 bis 4, **dadurch gekennzeichnet, daß** die Elektroden (35; 55) einen optisch betätigbaren Schalter umfassen, der im geschlossenen Zustand die Spannung als Stimulus (41) durchschaltet.

6. Retina-Implantat nach Anspruch 5, **dadurch gekennzeichnet, daß** der Schalter als Bereich einer weiteren Schicht (34) ausgebildet ist, die bei Bestrahlung mit dem nicht-sichtbaren Licht elektrisch nicht-leitend und bei Bestrahlung mit sichtbarem Licht elektrisch leitend ist.

7. Retina-Implantat nach Anspruch 6, **dadurch gekennzeichnet, daß** die weitere Schicht (34) aus einem amorphen, hydrogenisierten Silizium (a-Si:H) oder Legierungen desselben besteht.

8. Retina-Implantat nach Anspruch 5, **dadurch gekennzeichnet, daß** die Elektrode (55) als Kontaktfläche eine Schicht aus für das nicht-sichtbare Licht nicht-durchlässigem Material umfaßt.

9. Retina-Implantat nach einem oder mehreren der Ansprüche 1 - 8, **dadurch gekennzeichnet, daß** das nicht-sichtbare Licht Infrarot-Licht (25) ist.

10. Retina-Implantat nach einem oder mehreren der Ansprüche 1 - 9, **dadurch gekennzeichnet, daß** es als subretinales Implantat (13) ausgebildet ist.

11. Retina-Implantat nach einem oder mehreren der Ansprüche 1 - 10, **dadurch gekennzeichnet, daß** es für sichtbares Licht im wesentlichen durchlässig und als epiretinales Implantat (14) ausgebildet ist.

12. Retina-Implantat nach einem oder mehreren der Ansprüche 1 - 11, **dadurch gekennzeichnet, daß** es ein Substrat mit Durchgangsöffnungen aufweist.

13. Retina-Implantat nach Anspruch 12, **dadurch gekennzeichnet, daß** die Durchgangsöffnungen in einem starren Substrat vorgesehen sind.

14. Retina-Implantat nach Anspruch 12, **dadurch gekennzeichnet, daß** das Substrat flexibel oder als Netz aus einem flexiblen Trägermaterial ausgebildet ist.

## Claims

1. A retina implant (13, 14) having a surface (28) for applying same to a retina (12), whereby said surface (28) is provided with electrodes (35; 55) for stimulating cells (27) of said retina (12) and-whereby electrodes (35; 55) are driven by visible light (21') impinging on the retina (12) such that an electrical stimulus (41) is exertable on said cells (27) by said electrodes (35; 55), **characterized in that** said implant (13; 14) is provided with a photovoltaic layer (30; 50) effective for non-visible light (25'), and that said stimuli (41) are locally switchable by utilizing said voltage generated by the photovoltaic layer (30; 50).

2. The retina implant of claim 1, **characterized in that** said implant (13; 14) is provided on a large surface with said layer (30; 50).

3. The retina implant of claim 1 or claim 2, **characterized in that** in a top plan view said electrodes (35; 55) are surrounded by said layer (30; 50).

4. The retina implant of claim 3, **characterized in that** said electrodes (55) occupy about 30 % to 50 % of the surface of said layer (50).

5. The retina implant of anyone or several of claims 1 - 4, **characterized in that** said electrodes (35; 55) comprise a switch that is optically actuatable and feeds said voltage as stimulus (41) when being in a closed state.

6. The retina implant of claim 5, **characterized in that** said switch is configured as an area of a further layer (34), said further layer being electrically non-conductive when exposed to said non-visible light and being electrically conductive when exposed to said visible light.

7. The retina implant of claim 6, **characterized in that** said further layer (34) consists of amorphous hydrogenized silicon (a-Si:H) or alloys thereof.

8. The retina implant of claim 5, **characterized in that** said electrode (55) comprises as contact surface a layer of a material being impermeable for said non-visible light.

9. The retina implant of anyone or several of claims 1 - 8, **characterized in that** said non-visible light is infrared light (25).

10. The retina implant of anyone or several of claims 1 - 9, **characterized in that** it is configured as a sub-retinal implant (13).

11. The retina implant of anyone or several of claims 1 - 10, **characterized in that** it is substantially permeable for visible light and is configured as an epiretinal implant (14).

12. The retina implant of anyone or several of claims 1 - 11, **characterized in that** it comprises a substrate provided with through-openings.

13. The retina implant of claim 12, **characterized in that** the through-openings are provided in a rigid substrate.

14. The retina implant of claim 12, **characterized in that** the substrate is flexible or configured as a net made from a flexible carrier material.

## Revendications

1. Implant rétinien (13, 14) avec une surface (28) pouvant être appliquée sur la rétine (12), la surface (28) étant pourvue d'électrodes (35 ; 55) pour stimuler des cellules (27) de la rétine (12), les électrodes (35 ; 44) étant commandées par une lumière visible (21) parvenant sur la rétine (12), de manière que les électrodes (35 ; 55) exercent une stimulation électrique (41 ) sur les cellules (27), **caractérisé en ce que** l'implant (13 ; 14) est pourvu d'une couche photovoltaïque (30 ; 50) active pour une lumière non visible (25') et **en ce que** les stimuli (41) peuvent être commandés localement par exploitation de la tension produite par la couche photovoltaïque (30 ; 50).

2. Implant rétinien selon la revendication 1, **caractérisé en ce que** l'implant (13 ; 14) est pourvu de la couche (30 ; 50) sur une grande surface.

3. Implant rétinien selon la revendication 1 ou 2, **caractérisé en ce que** les électrodes (35 ; 55) sont entourées par la couche (30 ; 50) vue de dessus.

4. Implant rétinien selon la revendication 3, **caractérisé en ce que** les électrodes (55) occupent environ 10 % à 50 % de la surface de la couche (50).

5. Implant rétinien selon une ou plusieurs des revendications 1 à 4, **caractérisé en ce que** les électrodes (35 ; 55) comportent un interrupteur pouvant être actionné optiquement qui à l'état fermé laisse passer la tension en tant que stimulus (41).

6. Implant rétinien selon la revendication 5, **caractérisé en ce que** l'interrupteur est réalisé comme zone d'une autre couche (34) qui, lorsqu'elle est exposée à la lumière non visible, est électriquement non conductrice et lorsqu'elle est exposée à la lumière visible est électriquement conductrice.

7. Implant rétinien selon la revendication 6, **caractérisé en ce que** l'autre couche (34) est constituée d'un silicium amorphe, hydrogéné (a-Si:H) ou d'alliages de celui-ci.

8. implant rétinien selon la revendication 5, **caractérisé en ce que** l'électrode (55) comprend comme surface de contact une couche constituée d'une matière ne laissant pas passer la lumière non visible.

9. Implant rétinien selon une ou plusieurs des revendications 1 à 8, **caractérisé en ce que** la lumière non visible est une lumière infrarouge (25).

10. implant rétinien selon une ou plusieurs des revendications 1 à 9, **caractérisé en ce qu'**il est réalisé en tant qu'implant sous-rétinien (13).

11. Implant rétinien selon une ou plusieurs des revendications 1 à 10, **caractérisé en ce qu'**il est réalisé sensiblement perméable à la lumière visible et en tant qu'implant épirétinien (14).

12. Implant rétinien selon une ou plusieurs des revendications 1 à 11, **caractérisé en ce qu'**il comporte un substrat avec des orifices de passage.

13. Implant rétinien selon la revendication 12, **caractérisé en ce que** les orifices de passage sont prévus dans un substrat rigide.

14. Implant rétinien selon la revendication 12, **caractérisé en ce que** le substrat est réalisé flexible ou sous la forme d'un filet fait d'un matériau de support flexible.
